# EUROPEAN PATENT APPLICATION

(11) **EP 1 862 143 A1**
(43) Date of publication of application: **05.12.2007**
(21) Application number: 07009870.2
(22) Date of filing: 17.05.2007
(51) Int. Cl.: A61C 8/02

(54) **Method for the guided regeneration of bone and/or periodontal tissues in the medical surgical and dental field and device thus obtainable**

(30) Priority: 18.05.2006 IT MO20060159
(71) Applicant: Marchesi, Marcello, 41100 Modena (IT)
(72) Inventor: Marchesi, Marcello, 41100 Modena (IT)
(74) Representative: Brogi, Graziano

(57) **Abstract**

The method for the guided regeneration of bone and/or periodontal tissues in the medical surgical and dental field comprises the phases which consist in: acquiring information relating to the position, the conformation and the dimensions of an area of bone and/or periodontal tissues to be regenerated; processing the information by means of a software program implemented on a computer for the determination of the three-dimensional software model of a device that can be grafted in the area and suitable for supporting the regeneration of the tissues; making the device starting with the three-dimensional software model using a substantially automated rapid prototyping process; at least partially covering the device with an outer membrane suitable for inducing a compartmentation of the tissues.

The device for the guided regeneration of bone and/or periodontal tissues in the medical surgical and dental field comprises a substantially porous structure and at least a coating membrane of at least a portion of the outer surface of the structure.

## Description

The present invention refers to a method for the guided regeneration of bone and/or periodontal tissues in the medical surgical and dental field and the device thus obtainable.

It is known how often, in various surgical fields, the regeneration is required of bone lost due to atrophies, injuries and illnesses of various kinds, etc.

The grafts used thus far to regenerate bone have been of natural origin (autogenous, allogeneic and xenogeneic) or synthetic such as, among others, those in hydroxiapathite porcelain or tricalcic phosphate.

The grafts have often been covered with membranes for the purpose of producing tissue compartmentation and preventing the growth of soft tissues inside the bone structure. These membranes, in turn, are of natural or synthetic origin and are re-absorbable or non re-absorbable.

With the grafts have also been used both growth or modulation factors such as the BMP (Bone Morphogenetic Proteans), and precursor cell groups.

In the specifically dental sector, the need has arisen to regenerate bone and periodontal tissue around dental roots suffering from parodontitis.

In this case as well grafts, membranes and growth or modulation factors such as amelogenins have been used.

The field of tissue grafts continues to expand but to date many problems still remain to be solved.

The autogenous grafts create post-operation softness and the available material is limited, allogeneic and xenogeneic grafts fail to provide absolute reliability as regards the transmission of infective or immunologically active factors.

These materials are often available in granules or powders that are unable to withstand loads and which, on the contrary, risk being dislocated by even minimum pressure.

This condition obliges the granule material to be mainly used for retentive bone defects.

In the case of non-retentive bone defects, in order to preserve the placement of these granules, membranes are used (e.g., titanium reinforced) or surgical grilles.

Always in the case of non-retentive bone defects, cortico-medulla blocks have been used, but both the membranes and grilles and the blocks require an investigative adaptation procedure. The materials used are not personalised to the needs of the receiver site but must be modelled from time to time intra-operationally with a considerable lengthening of operating times.

To remedy such drawbacks a method is known for making grafts by means of rapid prototyping (RP).

Such method involves a first stage of determination of a three-dimensional model by means of a dedicated software programme (with the aid of a computer) and starting with a scanning operation or in any case with an image of the bone segment to be regenerated.

The data processed by the software programme generally include further information tied to the area of application of the graft and to the particular type of operation to be performed, such as the resistance of the graft to mechanical type stresses, the porosity of the graft to be made, the geometry and tortuousness of the porous structure.

A second and conclusive phase consists in the realisation of the graft starting with the three-dimensional model thus determined, using rapid prototyping procedures which involve, for example, the implementation of automated techniques by means of robotized equipment, such as the so-called direct techniques like SLS (Selective Laser Sintering), or indirect like DLP (Digital Light Projection) and ceramic gelcasting.

The graft made using one of the above methods is therefore shaped to precisely reproduce the outlines of the injured area for perfect adaptation to the receiver site, and features a structure able to withstand mechanical stresses.

The known methods for making grafts and the products thus obtainable are however susceptible to upgrading, in particular aimed at optimising the application for bone or tissue regeneration operations within the medical surgical and/or dental field.

In particular, such upgrading is suitable for preventing the undesired migration of soft tissues during the regeneration and, at the same time, for providing an adequate support for regeneration through the use of growth factors or modulators, precursor cell groups or other biologically active factors such as plasma enriched with platelets (PRP).

The main aim of the present invention is to provide a method for the guided regeneration of bone and/or periodontal tissues in the medical surgical and dental field that allows achieving the aforementioned upgrading.

Within the field of such technical aim, another object of the present invention is to cater for the above aims with a simple structure, of relatively practical implementation, safe to use and with effective operation, as well as having a relatively low cost.

The above objects are all achieved by the present method for the guided regeneration of bone and/or periodontal tissues in the medical surgical and dental field, comprising the phases which consist in:
- acquiring information relating to the position, the conformation and the dimensions of an area of bone and/or periodontal tissues to be regenerated;
- processing said information by means of a software program implemented on a computer, for the determination of the three-dimensional software model of a device that can be grafted in said area and suitable for supporting the regeneration of said tissues;
- making said device starting with said three-dimensional software model using a substantially automated rapid prototyping process;
characterized in that it comprises the at least partial covering of said device with an outer membrane suitable for inducing a compartmentation of said tissues.

Further characteristics and advantages of the present invention will appear even more evident from the detailed description of a preferred, but not exclusive, embodiment of a method for the guided regeneration of bone and/or periodontal tissues in the medical surgical and dental field and the device thus obtainable, illustrated indicatively by way of non limiting example, in the attached drawings wherein:
the figure 1 is a schematic view of an area of bone tissues to be regenerated;
the figure 2 is a transparent schematic and partial section view of a first embodiment of the device according to the invention, applied to the area shown in figure 1;
the figure 3 is a schematic and section view of an area of bone and periodontal tissues to be regenerated;
the figure 4 is a schematic and section view of a second embodiment of the device according to the invention, applied to the area shown in figure 3.

With special reference to such figures, a device for the guided regeneration of bone and/or periodontal tissues in the medical surgical and dental field, has been globally designated by reference number 1.

Advantageously, the method and the device 1 thus obtainable can be applied in different types of reconstruction operations.

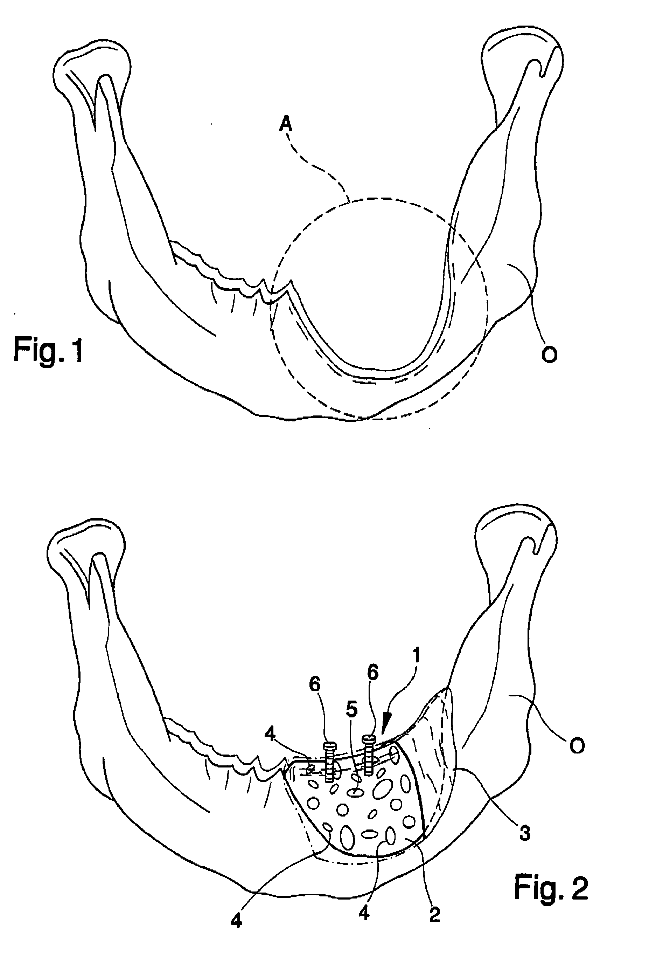

With particular, but not limitative reference to a first embodiment shown in the figures 1 and 2, the device 1 is applied for the regeneration of a jaw bone segment in an area A.

The device 1 comprises a substantially porous structure 2 and a membrane 3 which is arranged on a portion of surface of the device 1, placed in between the structure 2 and the soft tissues that cover the jaw.

Usefully, the device 1 can comprise a plurality of cavities 4, of the closed-cavity type, suitable for containing a compound 5 inductor or modulator of the growth of tissues and defined inside the structure 2.

In this case the compound 5 can be made up of bone morphogenic proteins (BMP) suitable for supporting the growth of the bone tissue O inside the porosity of the structure 2. Alternatively, the cavities 4 can contain blood (conveyed, for example, in hyaluronic acid swabs), autogenous bone tissues, plasma enriched with platelets (PRP) or other biologically active factors.

Advantageously, the device 1 can also have dental implants 6 or, in any case, can have one or more sites prepared for the installation of such implants.

Usefully, the device 1 can comprise fastening means for fastening to the jaw or maxillary bone segment to be regenerated, composed of one or more threaded elements, of the screw type or the like, which can be placed in between the structure 2 of the device 1 and the bone segment.

In particular, such screws can be fixed to the structure 2 and have respective protruding and threaded portions that can be screwed up in respective housings suitably prepared on the bone segment, in the area A to be regenerated.

Furthermore, the device 1 can be lined both inside and out with hyaluronic acid, in the form of gel, etc., suitable for favouring healing processes following the grafting of the device 1.

The method for making the device 1 comprises a first phase of acquisition of the information relating to the position, to the conformation and to the dimensions of the area A of bone tissues to be regenerated.

Usefully, the acquisition phase involves the taking of one or more images of the area A, for example, by means of a CAT scan or other procedures.

The image thus taken is then translated into data manageable by means of a suitable software program implemented on a computer (of the common personal computer type).

A second processing phase of the aforementioned data by means of the software program permits the determination of a three-dimensional software model of the device 1 which can be grafted in the area A and which is suitable for supporting the regeneration of the bone tissue O.

Usefully, the processed data can be integrated with parameters relating to the resistance to mechanical stresses, porosity and the particular shape of the porous structure of the device 1 to be made.

Furthermore, the processing phase of the three-dimensional software model of the device 1 permits defining the conformation and the arrangement of the membrane 3.

A third phase for making the structure 2 of the device 1 is performed starting with the processed software model and using a substantially automated rapid prototyping process, such as the so-called SLS (Selective Laser Sintering), DLP (Digital Light Projection) and ceramic gelcasting techniques.

The use cannot however be ruled out of other direct or indirect methods of making the structure 2.

In particular, the substantially porous structure 2 is obtained, for example, by sintering a material re-absorbable in the state of powders or granules, of the hydroxiapathite, tricalcic phosphate or other biocompatible material type.

The method comprises a fourth and last phase of at least partial covering of the structure 2 of the device 1 with the outer membrane 3, as defined during the processing phase, and pre-shaped to reproduce the profile of the outer surface of the structure 2.

The membrane 3 is made of synthetic re-absorbable material such as, for example, polylactic acid or polyglycolic acid.

Advantageously, the membrane 3 is placed in between the structure 2 and the soft tissues and is suitable for inducing a compartmentation of the tissues during the regeneration.

Particularly, the bone tissues O regenerate filling the porosities of the structure 2 of the device 1, while the soft tissues are kept outside the structure 2, separate from the membrane 3.

Advantageously, the method can comprise a supplementary processing phase by means of the software program of the above acquired data, for the definition of the three-dimensional software model of an apparatus for the controlled expansion of the soft tissues.

In particular, such apparatus is of the type of an expandable balloon installable in the area A to be regenerated and suitable for creating the space necessary for the subsequent insertion of the device 1.

The definition of this balloon by means of the software program makes it perfectly adaptable to the particular area A to be treated.

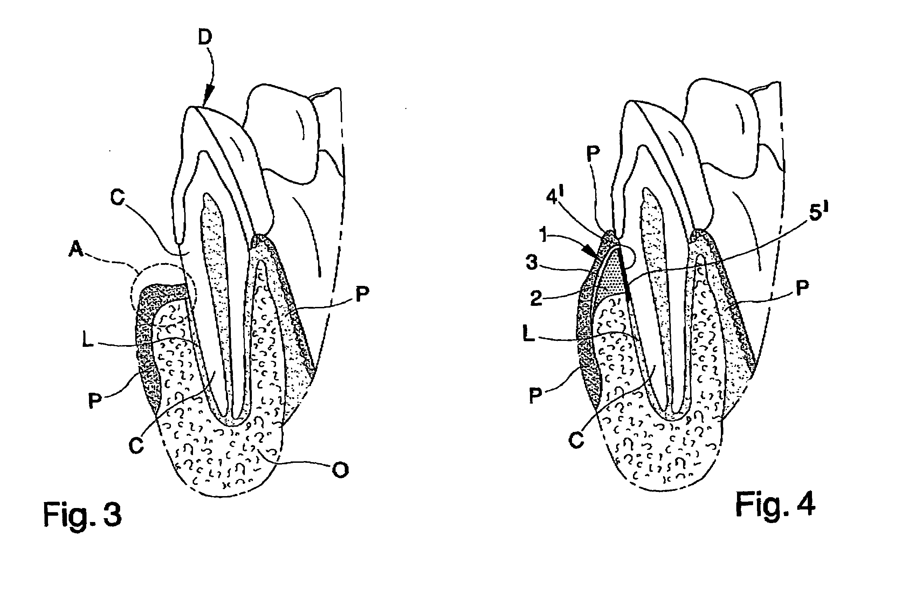

With particular but not limitative reference to a second embodiment, as schematically shown in the figures 3 and 4, the device 1 and the relevant method of manufacture can be used in the dental field, for example, in the case of pathologies such as periodontitis or the like.

In this case, the area A to be regenerated is defined by a portion of bone tissue O reabsorbed at the root C of a tooth D and by the corresponding soft tissues P.

Advantageously, the device 1 can comprise at least one cavity 4', of the open cavity type, which is suitable for containing a compound 5' inductor of tissue growth (growth and/or differentiation factor) and which is defined at a portion of the device 1 positionable in contact with the root C of the tooth D, inside the area A to be regenerated.

Usefully, the device 1 can comprise several cavities 4' for containing the above compound 5', in gel or other state, arranged facing the root C of the tooth D.

As figure 4 shows, the cavity 4' contains the compound 5' ensuring this adheres to the root C of the tooth D, so as to favour the regeneration of the periodontal ligament L. This compound 5' is made up of protein-based substances of the amelogenin type.

Advantageously, depending on the type of pathology and type of operation necessary, the device can be shaped differently and can be made up of several portions assembled together by interlocking.

In particular, two portions of the device 1 can be assembled together at the interstice defined between two adjacent teeth D, at the area to be regenerated A.

In practice it has been ascertained how the described invention achieves the proposed objects, and in particular, the fact is underlined that the presence of the membrane permits preventing the undesired migration of the soft tissues towards the inside of the structure, during the regeneration.

Furthermore, the presence of the cavities (open and superficial or closed and internal) for containing the growth or modulation factors, precursor cell groups, etc., provides an adequate support for the regeneration of the tissues.

The invention thus conceived is susceptible of numerous modifications and variations, all of which falling within the scope of the inventive concept.

Furthermore all the details may be replaced by other elements which are technically equivalent.

In practice, the contingent shapes and dimensions may be any according to requirements without because of this moving outside the protection scope of the following claims.

## Claims

1. Method for the guided regeneration of bone and/or periodontal tissues in the medical surgical and dental field, comprising the phases which consist in:
- acquiring information relating to the position, the conformation and the dimensions of an area of bone and/or periodontal tissues to be regenerated;
- processing said information by means of a software program implemented on a computer, for the determination of the three-dimensional software model of a device that can be grafted in said area and suitable for supporting the regeneration of said tissues;
- making said device starting with said three-dimensional software model using a substantially automated rapid prototyping process;
**characterized in that** it comprises the at least partial covering of said device with an outer membrane suitable for inducing a compartmentation of said tissues.

2. Method according to claim 1, **characterized in that** said processing comprises the defining of the conformation and the arrangement of said membrane.

3. Method according to one or more of the preceding claims, **characterized in that** said membrane is of the re-absorbable type.

4. Method according to one or more of the preceding claims, **characterized in that** said membrane is of the synthetic type.

5. Method according to one or more of the preceding claims, **characterized in that** said membrane comprises at least one between polylactic acid and polyglycolic acid.

6. Method according to one or more of the preceding claims, **characterized in that** said device comprises at least a containing cavity for containing a compound inductor or modulator of the growth of said tissue.

7. Method according to one or more of the preceding claims, **characterized in that** said containing cavity is an open cavity defined at a portion of said device positionable in contact with the root of at least a tooth by interposition of said compound inductor or modulator of the growth.

8. Method according to one or more of the preceding claims, **characterized in that** said containing cavity is a closed cavity defined inside said device.

9. Method according to one or more of the preceding claims, **characterized in that** said compound is protein-based.

10. Method according to one or more of the preceding claims, **characterized in that** said compound comprises at least one between amelogenin and bone morphogenic proteins (BMP).

11. Method according to one or more of the preceding claims, **characterized in that** said acquiring comprises the taking of at least one image of said area of bone and/or periodontal tissues to be regenerated.

12. Method according to one or more of the preceding claims, **characterized in that** said acquiring comprises the translating of said image taken into data manageable by means of said software program.

13. Method according to one or more of the preceding claims, **characterized in that** said device comprises a substantially porous structure.

14. Method according to one or more of the preceding claims, **characterized in that** said making comprises the obtaining of said structure by sintering of a material re-absorbable in the state of powders or granules.

15. Method according to one or more of the preceding claims, **characterized in that** said structure comprises at least one between hydroxiapathite and tricalcic phosphate.

16. Method according to one or more of the preceding claims, **characterized in that** said device comprises at least two portions which can be assembled together by interlocking.

17. Method according to one or more of the preceding claims, **characterized in that** the two portions of said device can be assembled together at the interstice defined between two adjacent teeth, at the area to be regenerated.

18. Method according to one or more of the preceding claims, **characterized in that** it comprises the preparing of said device for the installation of at least one dental implant.

19. Method according to one or more of the preceding claims, **characterized in that** it comprises the processing of said information by means of said software program, for the definition of the three-dimensional software model of an apparatus installable in said area and suitable for allowing the controlled expansion of the soft tissues.

20. Method according to one or more of the preceding claims, **characterized in that** said apparatus is of the balloon type or the like.

21. Method according to one or more of the preceding claims, **characterized in that** said device comprises fastening means for fastening to a bone segment in an area of bone and/or periodontal tissues to be regenerated.

22. Method according to one or more of the preceding claims, **characterized in that** said fastening means comprise at least one threaded element, of the screw type or the like, which can be placed in between said structure and said bone segment.

23. Method according to one or more of the preceding claims, **characterized in that** said threaded element can be fixed to said structure and has at least one protruding and threaded portion that can be screwed up in a respective housing suitably prepared on said bone segment, in said area to be regenerated.

24. Device for the guided regeneration of bone and/or periodontal tissues in the medical surgical and dental field, comprising a substantially porous structure, **characterized in that** it comprises at least a coating membrane of at least a portion of the outer surface of said structure.

25. Device according to claim 24, **characterized in that** said membrane is pre-shaped to reproduce the profile of the outer surface of said structure.

26. Device according to one or more of the claims from 24 and 25, **characterized in that** said membrane is of the re-absorbable type.

27. Device according to one or more of the claims from 24 to 26, **characterized in that** said membrane is of the synthetic type.

28. Device according to one or more of the claims from 24 to 27, **characterized in that** said membrane comprises at least one between polylactic acid and polyglycolic acid.

29. Device according to one or more of the claims from 24 to 28, **characterized in that** it comprises at least a containing cavity for containing a compound inductor of the growth of bone and/or periodontal tissues.

30. Device according to one or more of the claims from 24 to 29, **characterized in that** said containing cavity is an open cavity defined at a surface portion of said structure positionable in contact with the root of at least a tooth by interposition of said compound inductor or modulator of the growth.

31. Device according to one or more of the claims from 24 to 30, **characterized in that** said containing cavity is a closed cavity defined inside said structure.

32. Device according to one or more of the claims from 24 to 31, **characterized in that** said compound is protein-based.

33. Device according to one or more of the claims from 24 to 32, **characterized in that** said compound comprises at least one between amelogenin and bone morphogenic proteins (BMP).

34. Device according to one or more of the claims from 24 to 33, **characterized in that** said structure comprises at least one between hydroxiapathite and tricalcic phosphate.

35. Device according to one or more of the claims from 24 to 34, **characterized in that** it comprises at least two portions which can be assembled together by interlocking.

36. Device according to one or more of the claims from 24 to 35, **characterized in that** said two portions can be assembled together at the interstice defined between two adjacent teeth.

37. Device according to one or more of the claims from 24 to 36, **characterized in that** it comprises at least one site prepared for the installation of a dental implant.

38. Device according to one or more of the claims from 24 to 37, **characterized in that** it comprises fastening means for fastening to a bone segment in an area of bone and/or periodontal tissues to be regenerated.

39. Device according to one or more of the claims from 24 to 38, **characterized in that** said fastening means comprise at least one threaded element, of the screw type or the like, which can be placed in between said structure and said bone segment.

40. Device according to one or more of the claims from 24 to 39, **characterized in that** said threaded element can be fixed to said structure and has at least one protruding and threaded portion that can be screwed up in a respective housing suitably prepared on said bone segment, in said area to be regenerated.
